Europäisches Patentamt

**European Patent Office**

Office européen des brevets

(11) Publication number: **0 127 261**
**A1**

(12) **EUROPEAN PATENT APPLICATION**

(21) Application number: **84301242.8**

(22) Date of filing: **27.02.84**

(51) Int. Cl.³: **A 61 M 25/00**

(30) Priority: **30.03.83 US 480383**

(43) Date of publication of application:
**05.12.84 Bulletin 84/49**

(84) Designated Contracting States:
**DE FR GB IT**

(71) Applicant: **VANCE PRODUCTS INCORPORATED**
**1100 West Morgan Street**
**Spencer Indiana 47460(US)**

(72) Inventor: **Lawson, Russell K.**
**321 W. Nokomis Court**
**Fox Pointe Wisconsin 53226(US)**

(74) Representative: **Massey, Alexander et al,**
**MARKS & CLERK Scottish Life House Bridge Street**
**Manchester, M3 3DP(GB)**

(54) Method for providing percutaneous communication with the renal or ureteral cavities.

㊗ A method for providing percutaneous communication with the renal (15) or ureteral (14) cavities of a body. A catheter assembly (11) comprising a catheter (16) having a movable, controllable distal tip and a stylet (17) received within the catheter is directed into the ureteral passageway and there-through to a desired location in the ureteral or renal cavity. The distal tip is positioned to direct the stylet against the adjoining tissue (18) and the stylet is forced outwardly of the catheter and through the tissue to the exterior of the body.

./...

Croydon Printing Company Ltd

Fig.1

1

# METHOD FOR PROVIDING PERCUTANEOUS
# COMMUNICATION WITH THE RENAL OR URETERAL CAVITIES

## Background of the Invention

Field of the Invention: The present invention relates to the field of medical procedures for providing a percutaneous communicaticn with the renal and ureteral cavities, and more particularly to a method in which such communication is established by piercing through the skin from within such cavities.

Description of the Prior Art: Various procedures have been utilized to establish percutaneous communication with the renal and ureteral cavities. In most instances, these procedures have required that a stylet be advanced into the patient from the exterior, typically by either the trocar method or the needle method. These techniques therefore rely upon the operator's ability to precisely manipulate the stylet to the desired target. These procedures can be potentially dangerous unless great care is taken during the insertion to avoid over-penetration or misdirection into surrounding tissue.

A well known procedure has been the Seldinger technique, described in an article entitled "Catheter Replacement of Needle in Percutaneous Arteriography: New

Technique," S. I. Seldinger, Acta Radiologica, vol. 39, pp. 368-376 (1953). In accordance with this procedure, a stylet and obturated needle are introduced through the skin and extended into the desired area. The stylet is then removed and a wire guide is introduced through the needle. The needle is then removed and the tract is dilated by introducing over the wire guide successively larger dilators. The catheter is then introduced over the wire guide and the guide is removed, thereby providing communication with the internal location by way of the placed catheter.

Procedures have been developed for the placement of suprapubic catheters into the bladder by first establishing percutaneous communication with the bladder from within the bladder. These procedures entail different considerations since the bladder can be more readily inflated with liquid until it can be palpated through the skin, thus reducing the difficulties in making proper insertion of devices from outside of the body. Also, access to the interior of the bladder and control of equipment inserted into the bladder via the urethra is relatively simple because of the size and proximate location of the bladder to the urethra. Consequently, devices which are relatively straightforward in structure and operation can be designed for internal access and control in dealing with the bladder.

One technique for inserting suprapubic catheters involves inserting a rigid instrument having a curved distal portion that has a clamp thereon into the bladder and depressing the handle of the instrument to cause a protrusion in the suprapubic area. An incision is made from without at this point down to the tip of the distal portion of the rigid instrument, which is then brought through the incision. The clamp on the distal end is opened to accept and grasp a catheter, which is then withdrawn backwardly into the bladder. The clamp is released and the catheter is retained within the bladder while the rigid instrument is withdrawn through the urethra. This method can produce dangerous complications. For example, whenever a separate incision must be made there is always the attendant possibility of infection. This possibility is further increased in that the distal portion of the rigid instrument is first pushed out into the local environment and exposed to possible contamination, and is then drawn back into the bladder and out through the urethra.

Another suprapubic insertion technique is described in United States Patent No. 3,640,281, issued to Robertson on February 8, 1972. This technique includes several steps beginning with the insertion of a curved distal portion of a rigid instrument into the bladder through the urethra, the distal portion having a closure thereon. The bladder is inflated with a fluid through this instrument and the distal end of the instrument is engaged with the anterior wall of the bladder with sufficient force to provide a visible protrusion of the suprapubic region of the abdominal wall. An incision is made through the abdominal wall and the anterior wall of the bladder in register with the distal end of the instrument and the instrument is displaced outwardly through the incision. The closure is removed and a portion of a catheter is inserted into the distal end of the instrument, which is then withdrawn through the incision, leaving a portion of the catheter within the bladder. This technique also provides an increased possibility of contamination and infection as described for the previous procedure.

In United States Patent No. 3,920,023, issued to Dye et al. on November 18, 1975, there is described yet another procedure for placement of a suprapubic catheter. A hollow, curved and rigid tubing member is inserted transurethrally into the bladder. Because of the proximity of the bladder to transurethral access, and the simplicity of the physical shape and alignment of the bladder, a rigid, preshaped tubing member can be manipulated through the urethra and firmly into position against the interior wall of the bladder. A catheter having a cutting point at the distal end is received within the tubing member. The tubing member is manipulated to position its distal end against the anterior wall of the bladder in the suprapubic region and the cutting point is pushed through the tubing member to pierce the bladder wall and the skin. The cutting point is then pulled outwardly and removed from the catheter to leave a portion of the catheter within the bladder and to provide percutaneous communication with the bladder through the catheter.

Catheter placement units for use in establishing a suprapubic catheter system are also described in United States Patent Nos. 4,205,675, issued to Vaillancourt on June 3, 1980; 3,680,562, issued to Wittes et al. on August 1, 1972; and, 3,860,006, issued to Patel on January 14, 1975.

0127261

## Summary of the Invention

Briefly describing one aspect of the present invention there is provided a method for establishing percutaneous communication with the renal or ureteral cavities of a body, the method comprising inserting a catheter assembly successively through the urethra and the bladder and into the ureteral passageway. The catheter assembly includes a catheter having a movable, controllable tip portion and a stylet received within the catheter. The tip portion is further directed through at least a portion of the ureter or fully through the ureter and into the renal cavity. The catheter is manipulated to have its distal end positioned at a desired location for the percutaneous communication, and the stylet is then extended outwardly of the catheter and forced through the adjoining tissue to the exterior of the body.

It is an object of the present invention to provide a method for establishing percutaneous communication with the renal or ureteral cavities of a body.

It is a further object of the present invention to provide a method as described which entails a minimum of risk for infection.

Another object of the present invention is to provide a method which may be reliably performed, and does not involve difficulties of prior techniques regarding possible over-insertion or misdirection of devices directed from outside of the body.

Further objects and advantages of the present invention will become apparent from the description of the preferred embodiment which follows.

## Brief Description of the Drawings

FIG. 1 is a somewhat schematic view showing a catheter assembly inserted into the renal cavity with the stylet extending through the skin in accordance with the method of the present invention.

FIG. 2 is a plan view of the inner and outer catheters and stylet useful in accordance with the present invention.

FIGS. 3a and b are magnifications of the distal tip of the stylet useful with the present invention, showing two of many alternate configurations to facilitate passage of the tip through the surrounding tissue.

FIG. 4 is an assembly view showing a catheter assembly which is useful in the performance of the present invention.

FIG. 5 is a partial view of the assembly of FIG. 3, particularly showing the manner of controlling the direction of the distal tip of the inner catheter.

FIG. 6 is a plan view of an alternate form of a catheter assembly useful in the performance of the present invention.

FIGS. 7a-f are schematic views depicting steps performed in the method of the present invention.

## Description of the Preferred Embodiment

For the purposes of promoting an understanding of the principles of the invention, reference will now be made to the embodiment illustrated in the drawings and specific language will be used to describe the same. It will nevertheless be understood that no limitation of the scope of the invention is thereby intended, except in accordance with the language of the appended claims.

Referring in particular to the drawings, there is illustrated a method 10 for providing percutaneous communication with the renal or ureteral cavities of a body. The method utilizes a catheter assembly 11 which is inserted successively through the urethra 12 and the bladder 13, and at least into the ureteral cavity 14. As will be appreciated from the following description, the method contemplates that the assembly 11 is directed through at least a portion of the ureteral cavity 14, and in desired instances is inserted through the ureter and to a location within the renal cavity 15.

The catheter assembly 11 includes a hollow catheter 16 having a movable, controllable tip portion. The assembly further includes a stylet 17 received within the catheter. As opposed to other techniques for dealing with suprapubic catheter placements, the present invention provides for communication with either the ureteral or renal cavities. The movable, controllable tip portion permits the directing of the catheter assembly, including the stylet received therein, to be aimed and moved through the urethra and bladder and into and through the ureter to the desired location.

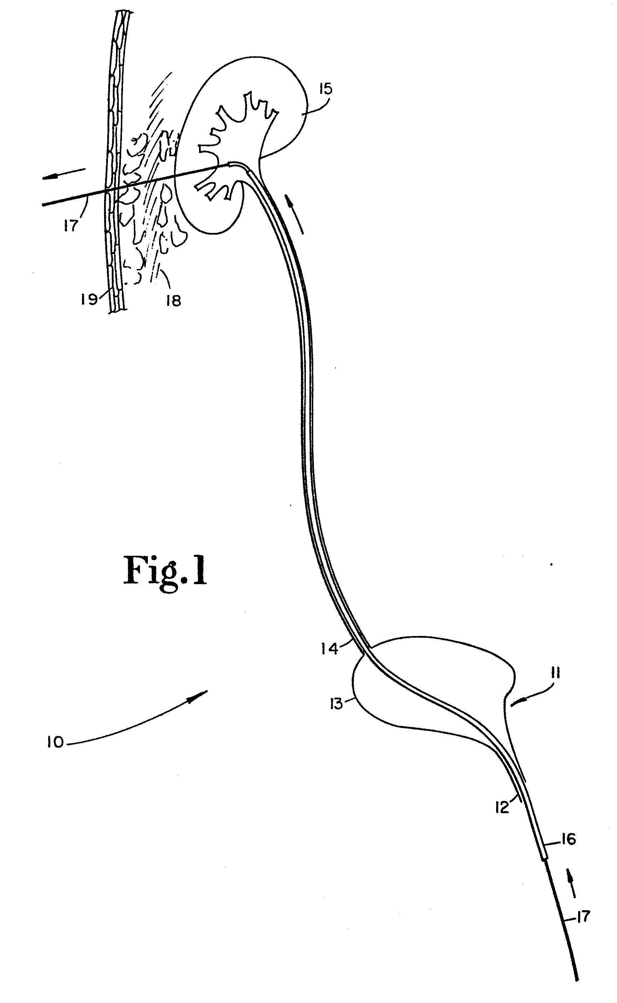

As depicted in FIG. 1, the catheter assembly is manipulated to the desired location and the stylet 17 is thereby positioned to be forced through the adjoining ureteral or renal wall and the surrounding tissue 18, and eventually through the skin 19 to the exterior of the body. Although not specifically shown, the procedure and physical positioning for extending the stylet through the ureteral wall is essentially the same as that depicted with respect to the renal wall.

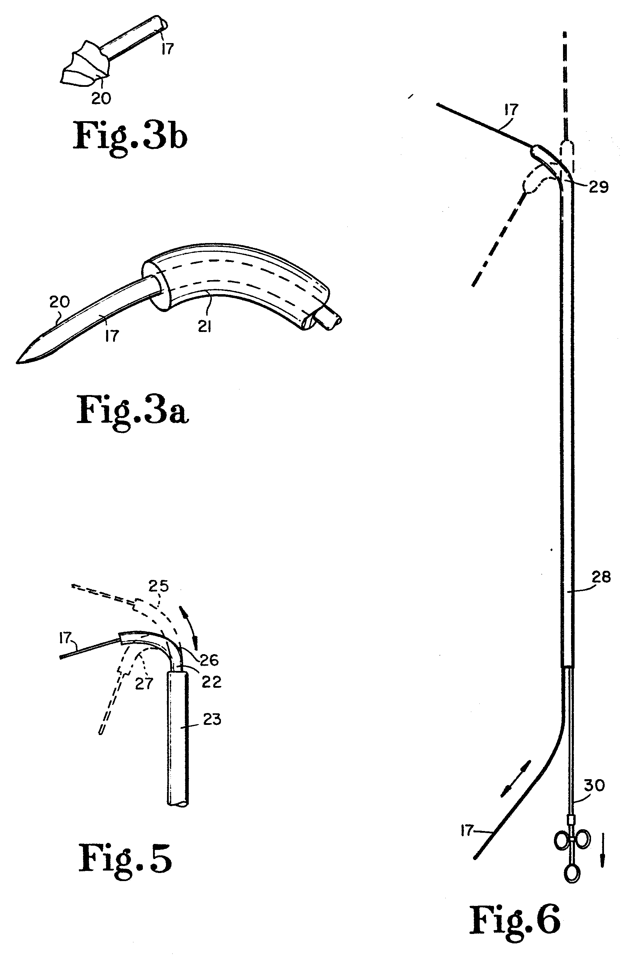

In a preferred construction, the catheter assembly 11 used in performing the method of the present invention includes a stylet 17 having a distal end 20 configured to facilitate passage of the tip through the adjoining tissue. As two alternate configurations there are shown in FIGS. 3a and b a needle point and a burr point, respectively. The needle point is suited to a direct forcing of the stylet through the tissue, whereas the burr point is more adapted to a drilling or boring of the stylet through the tissue. Either design, or others still, are useful for the method described herein.

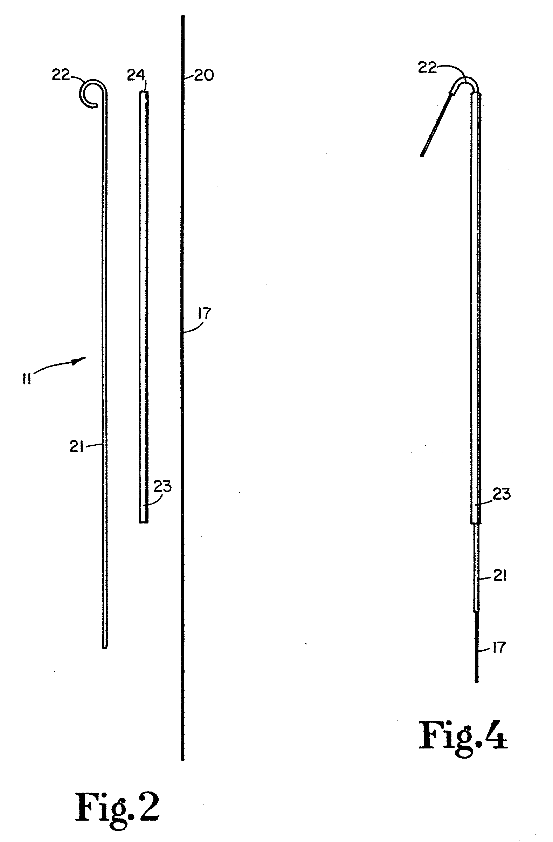

The movable, controllable tip portion of the catheter may be obtained by a variety of techniques. In the preferred construction, there is provided an inner, hollow catheter 21 within which the stylet 17 is received. The inner catheter has a distal tip 22 which has a preset coil (FIG. 2) which is sufficiently flexible as to be extended within a straight, outer catheter 23. The coiled portion is also sufficiently resilient that it will return to a coiled configuration upon being extended outwardly of the outer catheter, as shown particularly in FIG. 5.

The inner catheter 21 is received within the outer catheter 23 and cooperates therewith to direct the catheter assembly as it is moved through the various body passageways and cavities in accordance with the present invention. As the inner catheter is extended beyond the distal tip 24 of the outer catheter, the inner catheter will start to bend as shown by the first position 25 in FIG. 5. Further extension of the inner catheter relative the outer catheter will result in progressively greater bending of the inner catheter as it seeks to resume the preset, coiled configuration. This progressive bending of the inner catheter is represented by the positions 26 and 27 in FIG. 5. The inner catheter may similarly be retracted within the outer catheter, resulting in progressively lesser bending of the distal tip portion of the inner catheter.

In this manner, the catheter assembly is provided with a catheter having a movable, controllable tip portion which is directable through the various passageways and cavities in performing the method of the present invention. In particular, the present invention includes the directing of this tip portion through the urethra and bladder and into the ureteral cavity. Proper control of the deflecting of the movable tip portion permits the unit to be directed into the ureteral cavity and further enables the unit to be directed within the ureteral cavity to the renal cavity, as may be desired.

The catheter assembly is manipulated to locate the distal end 20 of the catheter 21 to a desired location within the ureteral or renal cavities. The open end of the distal tip of the catheter can be positioned and aimed toward a favorable location, thereby aiming the stylet in the same manner. The stylet 17 is then extended out of the catheter and through the adjoining tissue to the exterior of the body. The penetrating distal tip of the stylet, as illustrated in FIGS. 3a and b, will facilitate movement of the stylet through the tissue.

Extending the stylet through the tissue is accomplished by moving the stylet relative to the inner catheter. Force is applied to the stylet at a location spaced from the distal tip of the stylet, typically at the proximal end of the stylet which is accessible outside of the urethra. In one approach, the stylet is provided with a needle point and the tip is forced through the tissue by either a single, impactive stroke, or by a series of successive strokes. Alternatively, the stylet is provided with a burr tip and is forced through the tissue by rotatingly drilling or boring the tip through the tissue.

Alternate constructions may be employed for the catheter assembly useful with the present invention. As shown in FIG. 6, the assembly may include a double lumen catheter 28 which has a deflectable tip 29. The first passageway of the catheter is for receiving the stylet 17. The second passageway receives a deflecting handle unit 30, such as a Cohen deflecting handle, which is used to control the deflecting of the tip by varying the relative extension of an incorporated wire within the second lumen.

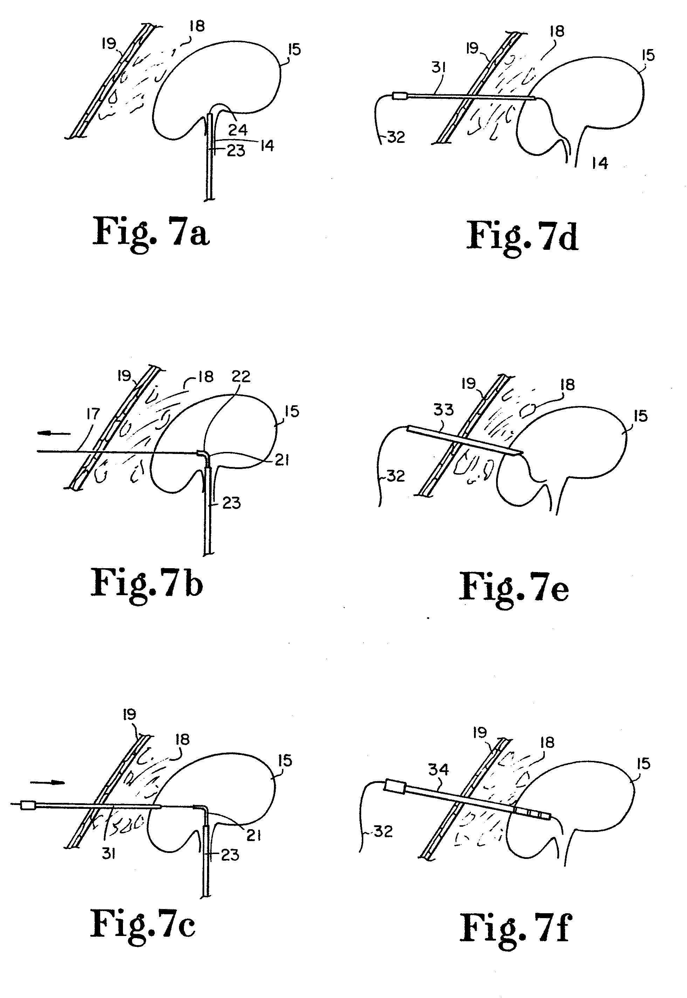

Extension of the stylet 17 to the exterior of the body provides a percutaneous communication with the ureteral or renal cavity at the location of the catheter assembly. Additional steps may be taken to establish the desired placement of equipment along the track established by the stylet. In a preferred procedure, a hollow needle 31 (FIG. 7c) is introduced over the stylet 17 and thereby extended into the cavity. The catheter is removed from the body through the bladder and urethra, and the stylet is also removed. A wire guide 32 is inserted through the hollow needle 31 and into the cavity, as shown in FIG. 7d.

With the wire guide 32 in place, the hollow needle 31 is removed from the wire guide. The track is then dilated by introducing over the wire guide a series of successively larger dilators (FIG. 7e). With the dilators removed, a catheter 34 (FIG. 7f) is introduced over the wire guide and the wire guide is thereafter removed. By this procedure, a catheter has been placed along the track of the stylet which initially extended out through the skin.

It will be appreciated that the described procedure substantially eliminates the risks attendant to prior art procedures in which a needle or trocar may be over inserted or misdirected. Since access is first established from within the ureteral or renal cavity, an accurate and controlled insertion of the needle from outside the body can be accomplished. This substantially reduces, and practically eliminates, the possibility of a misguided puncture resulting in immediate injury to critical structures or potentiating serious damage to tissue or organs during dilation or introduction of a catheter later in the procedure. For example, in the case of the renal cavity the present procedure permits an easier and more reliable avoidance of critical structure such as the five segmental arteries leading to the kidney from the renal artery. The stylet can be controllably directed through infundibulae or the deepest recesses of the calyces to avoid puncturing of the segmental arteries.

The catheter assembly utilized by the present invention can be fabricated in a variety of ways. The stylet may for example be formed of a polymeric, metallic or ceramic material. The stylet may be partly rigid and partly flexible, segmented, or a composite of several materials and constructions. Examples would include a glass fiber reinforced polymer or a metal point or leading segment backed and pushed by a more flexible polymer segment. The outer and inner catheters could be polymeric, metallic or ceramic, with exemplary materials including polyethylene, polypropylene, polyurethane, polycarbonate, polyvinylchloride or a stainless steel wire coil.

Access for the catheter assembly into the ureteral and renal cavities may be provided in one instance through the urethra and bladder. However, access could also be provided in partially open procedures as a result of a proximate opening to the bladder. The outer and/or inner catheters could be steerable by external/proximal control in order to deflect and direct the tip from which the stylet exits within the body. This concept could be employed through an er oscope, such as a cystoscope, panendoscope, choledocoscope, nephroscope, ureterorenoscope, arthroscope or the like, in order to establish another tract from within the space invaded by the endoscope.

While the invention has been illustrated and described in detail in the drawings and foregoing description, the same is to be considered as illustrative and not restrictive in character, it being understood that only the preferred embodiment has been shown and described and that all changes and modifications that come within the spirit of the invention are desired to be protected.

CLAIMS:

1. A method for providing percutaneous communication with the renal or ureteral cavities of a body, which method comprises the steps of:

a. inserting a catheter assembly into the ureteral passageway, the catheter assembly including a hollow catheter and a stylet received within the catheter, said catheter having a movable, controllable tip portion and having a distal end, said inserting comprising the directing of the tip portion of said catheter to insert the distal end of said catheter into the ureteral passageway;

b. directing the tip portion of said catheter through at least a portion of the ureteral passageway to locate the distal end of said catheter adjacent to a desired location in the ureteral or renal cavities, the location being that at which the percutaneous communication is desired; and

c. extending said stylet out of said catheter and through the adjoining tissue to the exterior of the body, said stylet being provided with a penetrating distal tip for extension through the adjoining tissue, said extending of said stylet comprising forcing the distal tip of said catheter through the adjoining tissue by moving said stylet relative said catheter by force applied to said stylet at a location spaced from the distal tip.

2. The method of claim 1 in which step a. includes inserting a catheter assembly successively through the urethra and the bladder and into the ureteral passageway.

3. The method of claim 2 in which step b. comprises directing the tip portion of said catheter through the length of the ureteral cavity and into the renal cavity to locate the distal end of said catheter adjacent the desired location in the renal cavity.

4. The method of claim 3 in which the penetrating tip of said stylet includes a needle point.

5. The method of claim 4 in which step c. comprises forcing the distal tip through the adjoining tissue in a single impactive stroke.

6. The method of claim 4 in which step c. comprises forcing the distal tip through the adjoining tissue in a series of successive impactive strokes.

7. The method of claim 3 in which the penetrating tip of said stylet includes a burr, and in which step c. comprises forcing the distal tip through the adjoining tissue by rotatingly drilling the tip through the tissue.

8. The method of claim 3 in which the·catheter assembly includes an inner, hollow catheter within which is received said stylet and an outer, hollow cathether within which is received said inner catheter, said inner catheter having a flexible, curved tip and being directable by extending and retracting said inner catheter relative said outer catheter to vary the extent of curve of the inner catheter, said directing of step b. comprising the controlling of the extension of said inner catheter relative said outer catheter to direct the tip of the inner catheter to the desired location, step c. comprising extending said stylet from said inner catheter by applying force to move said stylet relative said inner catheter.

9. The method of claim 8 in which step b. comprises directing the tip portion of said catheter through the length of the ureteral cavity and into the renal cavity to locate the distal end of said catheter adjacent the desired location in the renal cavity.

15

0127261

10. The method of claim 9 in which the penetrating tip of said stylet includes a needle point.

11. The method of claim 10 in which step c. comprises forcing the distal tip through the adjoining tissue in a single impactive stroke.

12. The method of claim 10 in which step c. comprises forcing the distal tip through the adjoining tissue in a series of successive impactive strokes.

13. The method of claim 9 in which the penetrating tip of said stylet includes a burr, and in which step c. comprises forcing the distal tip through the adjoining tissue by rotatingly drilling the tip through the tissue.

14. The method of claim 2 and which further includes, after step c., the following steps:
   d. introducing over said stylet from exterior of the body a hollow needle;
   e. moving said needle over said stylet to have the distal end of said needle within the ureteral or renal cavity;
   f. removing said catheter from the body through the bladder and urethra; and
   g. removing said stylet from the body.

15. The method of claim 14 and which further includes, after step g., the step h. of introducing a wire guide through the needle and extending into the ureteral or renal cavity.

16. The method of claim 15 and which further includes, after step h., the following steps:
   i. removing said needle from said wire guide;

j. dilating the opening through which the wire guide is received by introducing over said wire guide a series of successively larger dilators;

k. introducing over the wire guide a catheter; or one of various endoscopes professed and used in the state-of-the-art practice of endourology for calculi removal or surgical intervention; and

1. removing the wire guide or leaving a safety wire guide in place during endoscopic surgery.

0127261

Fig.1

0127261

22  24  20

17

11

21

23

Fig.2

22

23

21

17

Fig.4

0127261

**Fig.3b**

**Fig.3a**

**Fig.5**

**Fig.6**

Fig. 7a

Fig.7b

Fig.7c

Fig.7d

Fig.7e

Fig.7f

**0127261**

Application number

EP 84 30 1242

## DECLARATION

which under Rule 45 of the European Patent Convention shall be considered, for the purpose of subsequent proceedings, as the European search report

**European Patent Office**

| The Search Division considers that the present European patent application does not comply with the provisions of the European Patent Convention to such an extent that it is not possible to carry out a meaningful search into the state of the art on the basis of all claims.<br>Reason | CLASSIFICATION OF THE APPLICATION (Int Cl ) |
|---|---|
| Method for treatment of the human or animal body by surgery or therapy (see article 52 (4) of the European Patent Convention).<br><br>---- | A 61 M 25/00 |

| Place of search | Date of completion of the search | Examiner |
|---|---|---|
| The Hague | 14-06-1984 | GLAS |

EPO Form 1504  06.78